(19) Europäisches Patentamt / European Patent Office / Office européen des brevets

(11) **EP 4 446 734 A1**

(12) **EUROPEAN PATENT APPLICATION**

(43) Date of publication:
**16.10.2024 Bulletin 2024/42**

(21) Application number: **24163511.9**

(22) Date of filing: **14.03.2024**

(51) International Patent Classification (IPC):
**G01N 30/64** (2006.01)    **G01N 33/00** (2006.01)

(52) Cooperative Patent Classification (CPC):
**G01N 33/0031; G01N 30/64;** G01N 33/0047;
G01N 2030/642

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 LT 2023515**

(71) Applicant: **Volatile Technologies Lt, UAB
10224 Vilnius (LT)**

(72) Inventors:
• **Malaiska, Adomas Mindaugas
10224 Vilnius (LT)**
• **Lucas Luis, Lopez Serrano
10224 Vilnius (LT)**

(74) Representative: **Zaboliene, Reda
Metida
Business center Vertas
Gyneju str. 16
01109 Vilnius (LT)**

(54) **GAS DETECTOR SYSTEM AND ITS USE FOR THE IDENTIFICATION OF VOLATILE COMPOUNDS**

(57) The invention describes the method of separation and identification of volatile organic compounds, in which the airflow is passed through a detector system that includes a camera of photoionization sensors, which includes photoionization sensors that generate real time sensor responses and the resulting sensor responses, which are received and processed in a smart device, determining the specific concentrations of different volatile organic compounds in the sample. The invention describes a gas detector system for the separation and identification of volatile compounds, which includes a system of photoionization sensors and a smart device. The above system is able to distinguish various volatile organic compounds, identify them and provide specific concentrations of compounds. The said system can be placed in a portable device and can be used for a variety of purposes, including environmental monitoring, industrial process management, and safety and security.

Fig. 1

EP 4 446 734 A1

**Description**

The invention is related to the identification of the gas mixture using a gas detector system that includes at least two selective photoionization gas sensors connected to a smart device and an optional laboratory gas chromatography column.

TECHNICAL LEVEL

Typically, the selective identification and quantification of gas mixtures is carried out in the laboratory, and the analytical techniques of choice are gas chromatography and gas chromatography-mass spectrometry. In particular, gas chromatography, when combined with mass spectrometry, can be considered as the gold standard technique for the analysis and identification of the mixture of gas compounds and allows for an accurate separation of compounds, their identification by mass-charge ratio in the mass spectrometer and quantification.

The separation and identification of the gas mixture into constituent compounds is important when such compounds give the product of interest important properties. Such properties may include the aroma of food, which gives the desired taste qualities to food and which are usually determined by human tasters during the iterations to the production process; fermentation processes in which the quantification of methanol, ethanol and other released compounds can determine the efficiency of the fermentation process and optimize the synthesis of compounds for medicines and food production.

Laboratory analysis of gas mixtures is widely used in laboratories, but it would be beneficial to carry out the above analysis at the production site, for example, at the place of production of food or at the site of the fermentation process. To be able to conduct the analysis in question outside the laboratory, the typical laboratory requirements, such as a temperature-controlled room, trained laboratory technician or chemist, expensive consumables, including carrier gas, need to be overcome, thus achieving the benefits of reduced operating costs.

However, in today's production environment, there is no solution that can solve the separation, identification and quantification of volatile organic compounds (VOCs) in a complex mixture, for example, a smell from a food product or a fermentation tank.

Many systems exist that can be used as an external portable instrument for the detection, separation and quantification of volatile organic compounds, although all of them, as a rule, have operational difficulties and disadvantages. These systems use detectors such as metal oxide detectors, photoionization detectors, thermal conductivity detectors, flame ionization detectors, ion mobility spectrometers, electrochemical elements, polymer coatings, and so on.

A photoionization sensor is a type of gas sensor that is usually used to detect and measure the concentration of VOCs. The photoionization sensor acts on the gas sample with a source of ionizing energy, such as ultraviolet light or X-rays, and measures the resulting ionization current. The ionization current is proportional to the concentration of detectable VOCs and can be used to accurately measure the concentration of VOCs in the sample.

As one of the stages of analysis, chromatography is used, which is a separation method based on the different interaction of the sample with the stationary and mobile phase. Different compounds in the sample are released from the chromatographic column at different timestamp, depending on their affinity for the stationary and mobile phases. Measuring the elution time of different compounds allows to distinguish and identify them.

CN101308125A describes a photoionization detector and gas phase chromatography detection system, which includes a photoionization detector. The photo ionization detector includes a gas chamber, a stabilizer of the main state, an ultraviolet lamp, an excitation electrode, an assembly electrode and a housing.

US9651531 B2 describes a method and a system with the first and second sensors for comparing ammonia measurements in air samples. Air samples are taken from the vivarium environment to determine ammonia levels to control the airflow to the vivarium.

This patent describes only the determination of the level of ammonia using a different method of determination.

However, the ability of traditional sensors to distinguish and identify individual VOCs is limited if there are multiple VOCs in the sample. This limits the functionality of the sensors in cases where it is necessary to detect, measure and determine the presence of several compounds of the mixture.

BRIEF DESCRIPTION OF THE INVENTION

The invention describes the method of separation and identification of volatile organic compounds, in which the airflow is passed through a detector system that includes a chamber of photoionization sensors, which includes photoionization sensors that generate real time sensor responses and the resulting sensor responses are received and processed in a smart device, determining the specific concentrations of different volatile organic compounds in the sample. The invention describes a gas detector system for the separation and identification of volatile compounds, which includes a system of photoionization sensors and a smart device.

The method of separation and identification of volatile organic compounds involves the selection and placement of at least two sensors, each of which has a different range of response factors; the passage of a gas mixture by the supplied airflow through the said sensor chamber; receipt and processing of the received sensor response values at a smart device; calculation of the concentration of the compound in the gas mixture according to the response factors of the sensors.

Optionally, a chromatographic column can be used, in which case the gas mixture can be directed through it by the supplied airflow, for the separation of compounds in a mixture of at least several compounds.

Samples can be taken in two ways: 1) the headspace of the sample is collected in the sample vial and enters the sensor chamber using the supplied airflow; 2) the sample is injected into an air stream that enters directly into the sensor chamber using a gas syringe, a standard gas chromatographic method. Volatile compounds in mixtures are separated and each compound is quantified over a period of 1-10 minutes. Using this method, the specific concentration of the compound is determined.

The gas detector system is designed for the separation and identification of volatile compounds and includes a system of photoionization sensors and a smart device in which sensors, each of which has a different range of response factors, are configured to provide real time response values to a smart device. The smart device is configured to calculate the specific concentrations of volatile compounds on the basis of the response factors specific to each of the sensors over a period of 1-10 minutes.

The said system optionally includes a chromatographic column for the separation of compounds in the gas mixture.

The gas detector system is configured to operate using filtered ambient air, without requiring the preparation and supply of specific carrier gases used in lab setting.

The gas detector system is able to distinguish various volatile organic compounds, identify them and provide specific concentrations of compounds. The said system can be placed in a portable device and can be used for a variety of purposes, including environmental monitoring, industrial process management, and safety and security.

BRIEF DESCRIPTION OF THE DRAWINGS

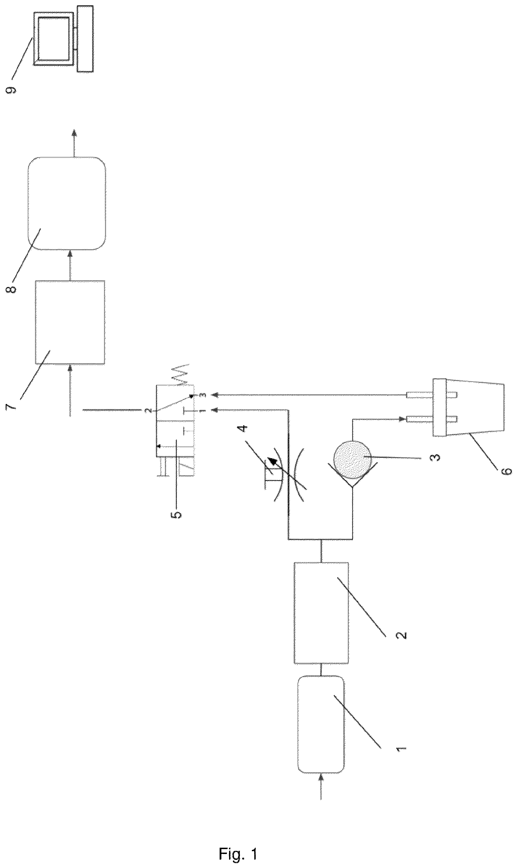

Figure 1. depicts the part of the gas detector system in which the separated compounds are passed through the sensor chamber.

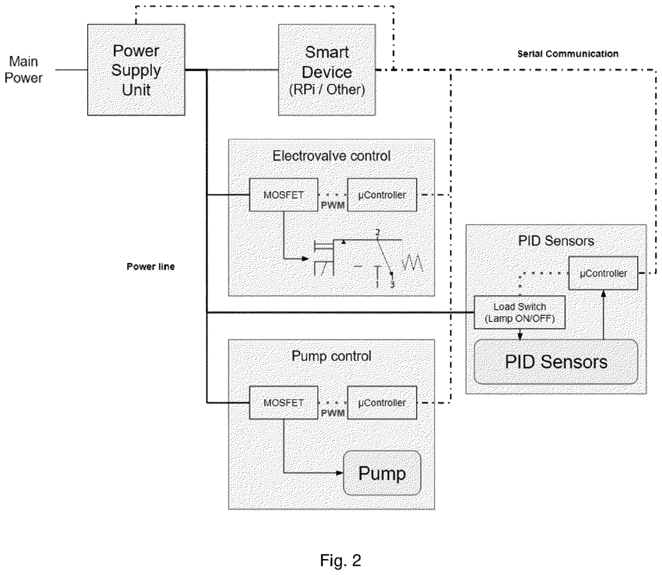

Figure 2. a diagram of the control unit is depicted.

DETAILED DESCRIPTION OF THE INVENTION

The gas detector system is designed for the separation, identification and quantification of volatile organic compounds.

The detector system includes taking gas samples from the sample flask, which is connected to a closed-loop airflow system, separating the compounds in the gas mixture, passing them through the gas sensor chamber and transmitting the signals of gas sensors to the smart device (9). The gas detector system includes a pump (1) for generating airflow, a moisture trap (2) for drying the incoming air, valves (3, 4, 5), a sample vial (6), a gas chromatography column (7) for compound separation, and a detector chamber (8) equipped with at least two or more gas sensors. The carrier gas used in the system is dried ambient air, which circulates with the help of a pump. The gas detector system includes several mechanisms for the separation and identification of compounds. The first is an optionally used chromatographic column (7), which separates gas compounds according to their polarity.

The second separation mechanism includes photoionization sensors of different charges. The gas detectors available on the market are of different ionization energies, such as 10.0, 10.6 and 11.7 eV (electronvolts). The energy of ionization determines the gas compounds that it can ionize and, in turn, detect. The lower the energy, the fewer different compounds can be detected. The higher ionization energy sensors can detect all the compounds that the lower-ionization energy version can detect, as well as some compounds previously not detected. For example, a 10.0 eV detector can detect 2.3-butanedione, which ionizes at 9.3-9.72 eV, but will not be able to detect ethanol, which ionizes at 10.4 eV. Therefore, in a mixture of 2,3-butanedione and ethanol, the 10.0 eV detector ionizes and detects only the 2,3-butanedione compound, while the 10.6 eV detector ionizes and detects both 2,3-butanedione and ethanol compounds.

The third mechanism for the separation of compounds includes a smart device that uses the response factors of different eV energy sensors to triangulate and assess the concentrations and presence of compounds. It is a known fact that sensors with different eV energies have fixed response factors given the same concentration of the gas compound. The response factors are typically expressed against a calibration with isobutylene gas as a multiplier number.

In the ethanol example, at 1 PPM (parts per million) of ethanol, it will not be ionized at all with a 10.0 eV detector. However, with a 10.6 eV detector, it will produce an 11 times lower response compared to the same concentration (1 PPM) of isobutylene. At the same time, the same 1 PPM ethanol produces only a 3 times lower response compared to 1 PPM isobutylene with an 11.7 eV detector. Differences in response factors can be used to triangulate the intensity of the response between different ionization energy sensors and in turn allow to identify the exact compound. As a result, using the known response factors allows to accurately quantify the concentration of the compounds. This method is described in detail in example 2.

The mechanisms described above give a surprisingly good result by separating complex gas mixtures into components and quantifying them. At the same time, such a system can be portable and easy to operate without special laboratory conditions, which provides significant operational benefits in an industrial or production environment or even in the laboratory as a rapid test instrument.

QUANTIFICATION OF A MIXTURE OF COMPOUNDS

Example 1

Yeast fermentation broth is used to synthesize biopharmaceutical compounds. In the process, glucose is injected into the broth and, depending on the parameters of the process, turns into ethanol, methanol, or both. The content of methanol and ethanol defines the quality of the process and is a good indicator if the final target compounds are formed in the broth. Assuming a specific case of such biopharmaceutical fermentation broth, gas phase components consist of two compounds of interest: ethanol and methanol.

Using current analytical methods, the final target compounds of the synthesis at the end of the fermentation process are screening using liquid chromatography - mass spectrometry. However, the process is expensive, time-consuming, solvents are used that can be harmful to the environment. It is also much less effective to find out the fermentation results only at the end of the process, whereas the insights obtained during the process would allow changing the parameters on the go and prevent discarding the test batch. There are several practical ways that allow observing the emerging ethanol and methanol in the intermediate time.

Sampling

We take 1-2 millilitres of the fermentation broth and incubate it in the sample vial at room temperature for 10 minutes. This ensures that the volatile substances in the sample, such as ethanol and methanol, evaporate to the empty headspace of the vial. Then we collect the measurements of headspace gas mixture in one of the two ways described below.

Sample measurements can be taken in two ways: 1) the sample headspace is collected in a vial and enters the sensor chamber using an airflow (8); 2) the sample is injected into an air stream which carries it directly into the sensor chamber (8) using a gas syringe, a typical gas chromatography method.

Primary result

The sample is selectively passed through the chromatography column (7) and enters a chamber of three sensors (8), each of which has a different eV energy: 10.0, 10.6 and 11.7. The chromatographic column (7) separates some compounds with a differential delay in time. The chromatographic column (7) may be also used to separate other compounds in the sample, e.g. urea and small residues. When a mixture of ethanol and methanol enters the sensor chamber (8), each of them reacts and creates peaks. However, the compounds of interest, ethanol, and methanol, are released through the column in a single peak and do not indicate an obvious separation in terms of time.

Each sensor generates an electrical signal expressed in millivolts (mV). Readings in mV are calibrated in advance based on the value of isobutylene PPM/PPB (parts per billion).

The concentration of a known compound or mixture of compounds is defined according to the following commonly known formula given by sensor manufacturers: RF = 1/[X1/RF1 + X2/RF2 + X3/RF3 ....], where RF is the total response factor and RF1, RF2, etc. are the response factors of each known compound. These specific response factors are provided by the sensor manufacturer for a list of various compounds, including ethanol, methanol, and many others.

The concentration displayed by the sensor is then adjusted to the response factor in order to correctly assess the specific concentration of the compound by multiplying the sensor output PPB/PPM by the total RF.

For example, when the readings of the 10.6 eV sensor indicate 100 PPB, but the response is to the ethanol compound, an ethanol response factor of 11 was used to obtain an adjusted PPB concentration (100 PPB x 11 = 1100 PPB).

It would normally be difficult to determine the concentration of a gas mixture if it is unknown, but using different sensors, each of which has a different range of response factors, concentrations can be determined mathematically.

Examples of calculations

The response factors were taken from publicly available information from manufacturers.

Table 1 - Sensor response factors (in parentheses is the name of the variable in the calculations)

| Compound | 10.0 eV sensor response factors | 10.6 eV sensor response factors | 11.7 eV sensor response factors |
|---|---|---|---|
| Ethanol | No response | 11 (RF2e) | 3 (RF1e) |
| Methanol | No response | No response | 2.9 (RF1m) |

In this example, the 10.6 sensor reacts only to ethanol and determines the concentration of ethanol. This is then used to determine the concentration of methanol according to the readings of the 10.0 sensor.
Isobutylene concentration to PPB calibration is sensor specific and must be done for each sensor separately.

Table 2 - Example of the generated sensor response

| | 10.0 eV sensor | 10.6 eV sensor | 11.7 eV sensor |
|---|---|---|---|
| Response mV above the baseline | 0 | 1.000 | 2.000 |
| Linking to the PPB | 0 | 10.000 | 60.000 |
| Metric value of PPB | S3 | S2 | S1 |

Since the 11.7 eV sensor reacts to both ethanol and methanol, the following equation of methanol and ethanol concentrations is compiled, where Cm = methanol concentration in PPB, Ce = concentration of ethanol in PPB, C%e = concentration of ethanol in the mixture, C%m = percentage concentration of methanol in the mixture:

$$1. \; Cm + Ce = S1 * RF1me$$

$$2. \; Cm + Ce = S1 * 1/[C\%m/RF1m + C\%e/RF1e]$$

$$3. \; (Cm + Ce) * (C\%m/RF1m + C\%e/RF1e) = S1$$

Since the 10.6 sensor reacts only to ethanol, the following equation of ethanol concentration is compiled:

$$4. \; Ce = S2 * RF2e$$

The percentage concentrations are 100 % and can also be expressed in PPB numbers:

$$5. \; C\%m = Cm/(Cm + Ce)$$

$$6. \; C\%e = Ce/(Cm + Ce)$$

Having changed equations 4-6 to 3, we get:

$$7. \; (Cm + S2 * RF2e) * ((Cm/(Cm + S2 * RF2e))/RF1m + \\ + ((S2 * RF2e)/(Cm + S2 * RF2e))/RF1e) = S1$$

Since the only unknown value is Cm, it can be solved using the values in Tables 1 and 2:

$$8.\ (Cm + 10000 * 11) * ((Cm / (Cm + 10000 * 11))/2.9 +$$
$$+ ((10000 * 11) / (Cm + 10000 * 11))/3) = 60000$$

After solving the equation, the following is obtained:

$$9.\ Cm = 67666{,}7\ \text{PPB}$$

$$10.\ Ce = 110000\ \text{PPB}$$

By adding more different ionization energy sensors that react differently, it is possible to further increase the number of different known compounds that we can distinguish from the mixture, identify, and quantify.

IDENTIFICATION OF AN UNKNOWN PEAK

Example 2

In other cases, it may be necessary to identify unknown peaks, especially when there is a high probability that the peaks do not coelute through the column, which means that each different compound creates its own separate peak.
Below is an example of a mixture of diacetyl and ethanol, in which we determine the peaks and quantify the concentrations. In this case, a gas chromatographic column (7) is used, which creates the separate peaks of these two compounds.
The response factors are taken from publicly available information from manufacturers.

Table 3 - the response factors of the sensor and their calculated ratios (in parentheses is the name of the variable in the calculations)

| Compound | 10.6 eV sensor response factors (S2) | 11.7 eV sensor response factors (S1) | Response factor ratio (S2/S1) |
|---|---|---|---|
| Ethanol | 11 (RF2e) | 3 (RF1e) | 3.67 |
| Diacetyl | 0.86 (RF2d) | 1 (RF1d) | 0.86 |

Since the compounds have stable ratios of response factors for a particular type of sensor, it is possible to calculate the ratio of response factors.
In turn, when observing the actual response of the sensor, the different reactions of different types of sensors can be compared with the ratio of response factors.

Table 4 - an example of the sensor response (in parentheses is given the name of the variable in the calculations)

| Acquired data | 10.6 eV sensor response (S2) | 11.7 eV sensor response (S1) | PPB ratio (S1/S2) |
|---|---|---|---|
| Peak 1 values in PPB | 1163 | 1000 | 0.86 |
| Peak 2 values in PPB | 2000 | 7333 | 3.67 |

In this example, peak 1 is identified as diacetyl and peak 2 as ethanol, based on matching the acquired PPB signal ratio to the calculated response factor ratio.
This method can be used with several mixtures of compounds when each of the compounds creates its own peak.
When we determine the peak of each compound, we can use the known values of the response factors to obtain a representative concentration of the compound. For example, where Ce = ethanol concentration in PPB, Cd = diacetyl concentration in PPB:

$$1. \quad Ce = S2 * RF2e = 2000 * 11 = 22000 \, \text{PPB}$$

$$2. \quad Cd = S2 * RF2d = 1163 * 0{,}86 = 1000 \, \text{PPB}$$

The presented studies show that the developed gas detector system can be used to isolate and identify and quantify various volatile organic compounds.

**Claims**

1. A method for the separation and identification of volatile organic compounds, **characterized in that** the method is comprised of selecting and placing in a sensor chamber at least two sensors, each having a different range of response factors; passing the mixture from the supply air stream through the said sensor chamber; reception and processing of received sensor responses in a smart device; calculation of the concentration of the compound in the gas mixture based on the response factors of the sensors.

2. A method for the separation and identification of volatile organic compounds according to claim 1, **characterized in that** after step a) an air stream is optionally passed through a chromatography column for separating the compounds.

3. The method for separation and identification of volatile organic compounds according to claim 1 or 2, **characterized in that** the volatile compounds in the mixtures are decomposed and each compound is quantified in 1-10 min. period.

4. A method for the separation and identification of volatile organic compounds according to any one of claims 1-3, **characterized in that** the specific concentration of the compound is determined.

5. A method for the separation and identification of volatile organic compounds according to any one of claims 1 to 4, **characterized in that** the sample is a mixture of various compounds.

6. A method for the separation and identification of volatile organic compounds according to any one of claims 1-5, **characterized in that** the sample is introduced from the headspace collected in the vial using an air stream.

7. A method for the separation and identification of volatile organic compounds according to any one of claims 1 to 5, **characterized in that** the sample is injected into the air stream.

8. A gas detector system for the separation and identification of volatile organic compounds, comprising a photoionization sensor system and a smart device, wherein the sensors, each having a different range of response factors, are configured to provide real time responses to the smart device, and the smart device is configured to calculate the specific concentrations of volatile compounds based on the response factors of each of the sensors during 1-10 min. period.

9. A gas detector system according to claim 8, wherein said system optionally includes a chromatography column for separating compounds.

10. A gas detector system according to claim 8 or 9, **characterized in that** said system is configured to operate using filtered ambient air without requiring the preparation and supply of a specific gas.

Fig. 1

Fig. 2

Europäisches
Patentamt

European
Patent Office

Office européen
des brevets

# EUROPEAN SEARCH REPORT

Application Number

EP 24 16 3511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MORONI DAVIDE ET AL: "Sensorized buoy for oil spill early detection", METHODS IN OCEANOGRAPHY, vol. 17, 5 November 2016 (2016-11-05), pages 221-231, XP093200383, ISSN: 2211-1220, DOI: 10.1016/j.mio.2016.10.002 Retrieved from the Internet: URL:https://pdf.sciencedirectassets.com/28 1499/1-s2.0-S2211122016X00035/1-s2.0-S2211 122016300123/main.pdf?X-Amz-Security-Token =IQoJb3JpZ2luX2VjEEEaCXVzLWVhc3QtMSJIMEYCI QDmMID0FHCsE%2BTZ2CNTNgDQQbp6nb5M2sTvWNbjY oSCCAIhAO359amqlOrCN670CVs6412LFLZiiiaElLz Z59DHyteaKrMFCFoQBRoMMDU5MDAzNTQ2ODY1IgyhU kttZ9IqG3Y> * chapter 3 and chapter 4 * | 1-8,10 | INV. G01N30/64 G01N33/00 |
| X | US 9 823 211 B1 (ALLEN DAN G [US]) 21 November 2017 (2017-11-21) * column 7, line 66 - column 8, line 6; figure 5a * * column 9, line 33 - line 38; figure 4 * * column 12, line 28 - line 56; figure 12 * | 1,4-7 | TECHNICAL FIELDS SEARCHED (IPC) G01N |

-/--

The present search report has been drawn up for all claims

| Place of search | Date of completion of the search | Examiner |
|---|---|---|
| Munich | 2 September 2024 | Gomez, Adriana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 24 16 3511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU HONGBO ET AL: "Rapid and sensitive detection of formaldehyde using portable 2-dimensional gas chromatography equipped with photoionization detectors", SENSORS AND ACTUATORS B: CHEMICAL, vol. 283, 29 November 2018 (2018-11-29), pages 182-187, XP093200572, NL ISSN: 0925-4005, DOI: 10.1016/j.snb.2018.11.156 Retrieved from the Internet: URL:https://pdf.sciencedirectassets.com/271353/1-s2.0-S0925400518X00252/1-s2.0-S0925400518321129/main.pdf?X-Amz-Security-Token=IQoJb3JpZ2luX2VjEGUaCXVzLWVhc3QtMSJGMEQCIDTiGEdcx3N2TdQNCzfAiqh+pYc3ZE6qqX4dEPQ8hXtQAiBaDhJYxw94spsH5Ad54OOJYOVXeQDjmeLt/sy/4VaJeyqzBQh+EAUaDDA1OTAwMzU0Njg2NSIMsrsIWiQstx4MQQr5K> * chapter 3 and chapter 4 * | 1,2,4-9 | |
| | ----- | | TECHNICAL FIELDS SEARCHED (IPC) |

The present search report has been drawn up for all claims

| Place of search | Date of completion of the search | Examiner |
|---|---|---|
| Munich | 2 September 2024 | Gomez, Adriana |

EPO FORM 1503 03.82 (P04C01)

EP 4 446 734 A1

## ANNEX TO THE EUROPEAN SEARCH REPORT
## ON EUROPEAN PATENT APPLICATION NO.

EP 24 16 3511

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2024

| Patent document cited in search report | | Publication date | Patent family member(s) | Publication date |
|---|---|---|---|---|
| US 9823211 | B1 | 21-11-2017 | NONE | |

For more details about this annex : see Official Journal of the European Patent Office, No. 12/82

EPO FORM P0459

12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101308125 A
- US 9651531 B2